# EUROPEAN PATENT APPLICATION

(11) **EP 4 098 277 A1**
(43) Date of publication of application: **07.12.2022**
(21) Application number: 21747862.7
(22) Date of filing: 29.01.2021
(51) Int. Cl.: A61K 39/395, A61K 47/26, A61K 47/18, A61K 47/22, A61K 47/12, A61P 35/00, C07K 16/28

(54) **STABLE ANTI-PD-1 ANTIBODY PHARMACEUTICAL PREPARATION**

(30) Priority: 30.01.2020 KR 20200011353
(71) Applicant: Samsung Bioepis Co., Ltd., Yeonsu-gu Incheon 21987 (KR)
(72) Inventor: JUNG, Young Seok, Incheon 22008 (KR); HONG, Ja Hye, Incheon 21982 (KR); JOO, Kyung Hee, Incheon 21982 (KR); HA, Young Wook, Incheon 21982 (KR); OH, In Young, Incheon 21982 (KR); KIM, In Ae, Seoul 05090 (KR)
(74) Representative: Boult Wade Tennant LLP
(86) International application number: PCT/KR2021/001210
(87) International publication number: WO 2021/154027

(57) **Abstract**

The present disclosure relates to a stable anti-PD-1 antibody pharmaceutical formulation and a method for preparing the same, wherein the pharmaceutical formulation includes: an anti-PD-1 antibody or an antigen binding fragment thereof; a stabilizer; and a buffer, and does not include a surfactant.

## Description

### TECHNICAL FIELD

The present disclosure relates to stable anti-PD-1 antibody pharmaceutical formulations and preparation methods thereof.

### BACKGROUND ART

To enhance pharmaceutical stability, there is an increasing awareness for the need to develop stable anti-PD-1 antibody pharmaceutical formulations. An anti-PD-1 antibody pharmaceutical formulation includes a buffer, a stabilizer, and a surfactant to attain pharmaceutical stability.

However, a surfactant like polysorbate is known to be decomposed through oxidation and hydrolysis, and reactive oxygen species (ROS) may be produced as a result, which may cause oxidative damage of antibody proteins.

Accordingly, there remains a need for the development of formulations capable of stabilizing antibody proteins even without including a surfactant, which may potentially affect the stability of an anti-PD-1 antibody pharmaceutical formulation.

### DESCRIPTION OF EMBODIMENTS

### TECHNICAL PROBLEM

An aspect provides a stable anti-PD-1 antibody pharmaceutical formulation comprising: (a) an anti-PD-1 antibody or an antigen binding fragment thereof; (b) a stabilizer; and (c) a buffer, wherein the pharmaceutical formulation does not comprise a surfactant and has a pH of about 4.5 to about 6.5.

Another aspect provides a method for treating a cancer, comprising administering the pharmaceutical formulation to a subject.

Still another aspect provides a method for preparing the pharmaceutical formulation.

### SOLUTION TO PROBLEM

All technical terms used herein, unless defined otherwise, have the same meanings as those generally understood by one of ordinary skill in the art to which the present disclosure belongs. Further, although methods or samples are described herein, those similar or equivalent thereto are also incorporated in the scope of the present disclosure. The numerical values described herein are considered to include the meaning of "about", unless otherwise specified. The contents of all the publications disclosed as references herein are incorporated in the present disclosure.

An aspect provides a stable anti-PD-1 antibody pharmaceutical formulation comprising: (a) an anti-PD-1 antibody or an antigen binding fragment thereof; (b) a stabilizer; and (c) a buffer, wherein the pharmaceutical formulation does not comprise a surfactant and has a pH of about 4.5 to about 6.5.

Unlike generally known pharmaceutical formulations comprising an antibody, the pharmaceutical formulation does not comprise a surfactant, but a desired environment for preservation of an anti-PD-1 antibody may be provided to maintain the stability. The pH of the pharmaceutical formulation may be, for example, pH 4.5 to pH 6.5, pH 4.5 to pH 6.3, pH 4.8 to pH 6.3, pH 5 to pH 6.3, pH 5.2 to pH 6.3, pH 4.5 to pH 6.0, pH 4.8 to pH 6.0, pH 5.0 to pH 6.0, pH 5.2 to pH 6.0, pH 4.5 to pH 5.8, pH 4.8 to pH 5.8, pH 5.0 to pH 5.8, pH 5.2 to pH 5.8, pH 4.5 to pH 5.6, pH 4.8 to pH 5.6, pH 5.0 to pH 5.6, pH 5.2 to pH 5.6, pH 4.9 to pH 5.5, pH 4.9, pH 5.0, pH 5.1, pH 5.2, pH 5.3, pH 5.4, or pH 5.5.

The term "antibody" refers to any type of antibody having the activity to specifically bind to PD-1. The antibody includes a monoclonal antibody, a polyclonal antibody, a humanized antibody, a human antibody and a chimeric antibody. The antibody may be a pembrolizumab.

The phrase "the pharmaceutical formulation not comprising an ingredient A", as used herein may mean that the pharmaceutical formulation does not include an ingredient A or substantially does not include an ingredient A. The phrase "substantially does not include an ingredient A" may be interpreted to include cases that an ingredient A is not included at all, A is present in a trace amount that cannot substantially affect features of the pharmaceutical formulation in any way, or A is present in an amount impossible to detect.

The term "antigen binding fragment" is a fragment that is capable of binding to a PD-1 antigen in an anti-PD-1 antibody, including, for example, Fab fragment, F(ab')₂ fragment, Fc fragment, or scFv fragment.

A "pembrolizumab " is a humanized antibody used in cancer immunotherapy and is currently marketed under the trade name KEYTRUDA^{®} which is one of several trade names. Pembrolizumab may be used in treating melanoma, lung cancer including non-small cell lung cancer (NSCLC), head and neck cancer including head and neck squamous cell cancer (HNSCC), Hodgkin lymphoma including classical Hodgkin lymphoma (cHL), urothelial cancer, renal cell carcinoma, stomach cancer, microsatellite instability-high cancer (MSI-H), mismatch repair deficient (dMMR) solid cancer, cervical cancer, liver cancer, Merkel cell carcinoma (MCC), or the like. The pembrolizumab may also include biosimilars or biobetters of active pembrolizumab present in commercially available KEYTRUDA products. The pembrolizumab may include a heavy chain complementarity-determining region (CDR)1 having an amino acid sequence of SEQ ID NO: 1, a heavy chain CDR2 having an amino acid sequence of SEQ ID NO: 2, a heavy chain CDR3 having an amino acid sequence of SEQ ID NO: 3, a light chain CDR1 having an amino acid sequence of SEQ ID NO: 4, a light chain CDR2 having an amino acid sequence of SEQ ID NO: 5, and a light chain CDR3 having an amino acid sequence of SEQ ID NO: 6. The pembrolizumab may include a heavy chain variable region having an amino acid sequence of SEQ ID NO: 7, and a light chain variable region having an amino acid sequence of SEQ ID NO: 8.

Pembrolizumab targets a programmed cell death protein 1 (PD-1) receptor of a lymphocyte. Pembrolizumab is an IgG₄ iso-type antibody allowing the immune system to kill cancer cells by blocking the protection mechanism of cancer cells. Pembrolizumab was approved for medical use in the United States in 2014. In addition, pembrolizumab was approved for treatment of unresectable or metastatic solid tumor having certain genetic anomalies, for example, mismatch repair deficiency or microsatellite instability, in 2017. Further, pembrolizumab may be used alone or in combination with other chemical therapeutic agents. Pembrolizumab may be administered by intravenous or subcutaneous injection.

Pembrolizumab may be produced by a general method that is known in the related art. For example, US Patent 9,834,605 and WO2008/156712A1 disclose methods which those skilled in the art may use in producing a pembrolizumab. The methods are herein incorporated by reference in the present disclosure. For example, pembrolizumab may be produced by recombinant expression of immunoglobulin light and heavy chain genes in the host cell.

A concentration of the anti-PD-1 antibody, for example, a pembrolizumab, or an antigen binding fragment thereof, may be a therapeutically effective amount for treatment of a cancer. In addition, the concentration of the anti-PD-1 antibody, for example, a pembrolizumab, or an antigen binding fragment thereof, may be selected in consideration of the stability, viscosity, etc. of the pharmaceutical formulation. The concentration of the anti-PD-1 antibody, for example, a pembrolizumab, or an antigen binding fragment thereof, may be, for example, 5 mg/mL to 300 mg/mL, 5 mg/mL to 250 mg/mL, 5 mg/mL to 200 mg/mL, 10 mg/mL to 200 mg/mL, 10 mg/mL to 165 mg/mL, 15 mg/mL to 160 mg/mL, 5 mg/mL to 45 mg/mL, 10 mg/mL to 40 mg/mL, 15 mg/mL to 35 mg/mL, 20 mg/mL to 30 mg/mL, 130 mg/mL to 170 mg/mL, 135 mg/mL to 165 mg/mL, 140 mg/mL to 160 mg/mL, 145 mg/mL to 155 mg/mL, about 25 mg/ml, or about 150 mg/ml. The anti-PD-1 antibody, for example, a pembrolizumab, or an antigen binding fragment thereof, may contribute to the stability of the pharmaceutical formulation and maintenance of the viscosity and pH suitable for administration at the concentrations listed above.

The stabilizer may be one or more selected from the group consisting of polyols, amino acids, and metal salts. As used herein, the term "polyol" refers to an excipient having multiple hydroxyl groups. The polyol may include a sugar, a sugar alcohol, or a sugar acid. The sugar refers to a soluble carbohydrate. The sugar may be a monosaccharide, a disaccharide, an oligosaccharide, or a polysaccharide. The sugar alcohol is an organic compound derived from sugar, and each carbon atom thereof has a hydroxyl group. The sugar acid refers to a sugar having a carboxyl group at one or both side(s) of the chain. The polyol includes an anhydride or a hydrate of a polyol. For example, trehalose may include trehalose dehydrate as well as trehalose.

The polyol may be one or more selected from the group consisting of glucose, fructose, mannose, galactose, sucrose, lactose, maltose, trehalose, mannitol, sorbitol, and polyethylene glycol. The polyol may be sorbitol, sucrose, trehalose, mannose, maltose, mannitol, or a mixture thereof. A concentration of the polyol may be freely adjusted within a desirable range for maintaining the stability of an anti-PD-1 antibody, for example, a pembrolizumab, or an antigen binding fragment thereof in the pharmaceutical formulation, and for maintaining the viscosity of the pharmaceutical formulation, and may individually vary depending on the specific type of polyols, sugar alcohols or sugar acids.

A concentration of the sugar may be 1.0%(w/v) to 15.0 %(w/v), 3.0 %(w/v) to 15.0 %(w/v), 5.0 %(w/v)to 15.0 %(w/v), 7.0 %(w/v)to 15.0 %(w/v), 1.0 %(w/v) to 10.0 %(w/v), 3.0 %(w/v) to 10.0 %(w/v), 4.0 %(w/v) to 10.0 %(w/v), 5.0 %(w/v) to 10.0 %(w/v), 7.0 %(w/v) to 10.0 %(w/v), 3.0 %(w/v) to 7.0 %(w/v), 4.0 %(w/v) to 7.0 %(w/v), 1.0 %(w/v) to 5.0 %(w/v), 2.0 %(w/v) to 5.0 %(w/v), 3.0 %(w/v) to 5.0 %(w/v), 4.0 %(w/v) to 5.0%(w/v), 4.5 %(w/v) to 5.0 %(w/v) (e.g., about 4.7 %(w/v)), 6.5 %(w/v) to 8.5 %(w/v) (e.g., about 6.8 %(w/v), about 7.0 %(w/v), about 7.2 %(w/v)), or 7.8 %(w/v) to 8.2 %(w/v) (e.g., about 7.8 %(w/v), about 7.9 %(w/v), about 8.0 %(w/v), about 8.1 %(w/v), or about 8.2 %(w/v)). The sugar may be, for example, sucrose, glucose, galactose, maltose, fructose, trehalose, or a mixture thereof.

A concentration of the sugar alcohol may be 1.0 %(w/v) to 20.0 %(w/v), for example, 1.0 %(w/v) to 15.0 %(w/v), 1.0 %(w/v) to 10.0 %(w/v), 2.5 %(w/v) to 10.0 %(w/v), 3.0 %(w/v) to 10.0 %(w/v), 3.5 %(w/v) to 10.0 %(w/v), 4.0 %(w/v) to 10.0 %(w/v), 1.0 %(w/v) to 8.0 %(w/v), 2.5 %(w/v) to 8.0 %(w/v), 3.0 %(w/v) to 8.0 %(w/v), 3.5 %(w/v) to 8.0 %(w/v), 4.0 %(w/v) to 8.0 %(w/v), 1.0 %(w/v) to 6.0 %(w/v), 2.5 %(w/v) to 6.0 %(w/v), 3.0 %(w/v) to 6.0 %(w/v), 3.5 %(w/v) to 6.0 %(w/v), 4.0 %(w/v) to 6.0 %(w/v), 4.0 %(w/v) to 5.5 %(w/v), or 4.0 %(w/v) to 5.0 %(w/v). The sugar alcohol may be, for example, sorbitol, mannitol, a hydrate thereof, or a mixture thereof.

The amino acid may be glycine, alanine, valine, leucine, isoleucine, proline, phenylalanine, tyrosine, tryptophan, serine, threonine, cysteine, methionine, asparagine, glutamine, lysine, arginine, histidine, aspartic acid, glutamic acid, a pharmaceutically acceptable salt thereof, or a mixture thereof. The amino acid may be, for example, glycine, proline, phenylalanine, tyrosine, tryptophan, lysine, arginine, a pharmaceutically acceptable salt thereof, or a mixture thereof.

A concentration of the amino acid as the stabilizer may be in a range of 0.1 mM to 300.0 mM, 0.5 mM to 300.0 mM, 1.0 mM to 300.0 mM, 5.0 mM to 300.0 mM, 10.0 mM to 300.0 mM, 25.0 mM to 300.0 mM, 30.0 mM to 300.0 mM, 50.0 mM to 300.0 mM, 80.0 mM to 300.0 mM, 100.0 mM to 300.0 mM, 120.0 mM to 300.0 mM, 0.1 mM to 250.0 mM, 0.5 mM to 250.0 mM, 1.0 mM to 250.0 mM, 5.0 mM to 250.0 mM, 10.0 mM to 250.0 mM, 25.0 mM to 250.0 mM, 30.0 mM to 250.0 mM, 50.0 mM to 250.0 mM, 80.0 mM to 250.0 mM, 100.0 mM to 250.0 mM, 120.0 mM to 250.0 mM, 0.1 mM to 200.0 mM, 0.5 mM to 200.0 mM, 1.0 mM to 200.0 mM, 5.0 mM to 200.0 mM, 10.0 mM to 200.0 mM, 25.0 mM to 200.0 mM, 30.0 mM to 200.0 mM, 50.0 mM to 200.0 mM, 80.0 mM to 200.0 mM, 100.0 mM to 200.0 mM, 120.0 mM to 200.0 mM, 0.1 mM to 160.0 mM, 0.5 mM to 160.0 mM, 1.0 mM to 160.0 mM, 5.0 mM to 160.0 mM, 10.0 mM to 160.0 mM, 25.0 mM to 160.0 mM, 30.0 mM to 160.0 mM, 50.0 mM to 160.0 mM, 80.0 mM to 160.0 mM, 100.0 mM to 160.0 mM, 120.0 mM to 160.0 mM, 130.0 mM to 150.0 mM, 0.1 mM to 100.0 mM, 0.5 mM to 100.0 mM, 1.0 mM to 100.0 mM, 5.0 mM to 100.0 mM, 10.0 mM to 100.0 mM, 25.0 mM to 100.0 mM, 30.0 to 100.0 mM, 50.0 to 100.0 mM, 80.0 to 100.0 mM, 0.1 to 50.0 mM, 0.5 to 50.0 mM, 1.0 mM to 50.0 mM, 5.0 mM to 50.0 mM, 10.0 mM to 50.0 mM, 25.0 mM to 50.0 mM, 30.0 mM to 50.0 mM, 0.1 mM to 40.0 mM, 0.5 mM to 40.0 mM, 1.0 mM to 40.0 mM, 5.0 mM to 40.0 mM, 10.0 mM to 40.0 mM, 25.0 mM to 40.0 mM, 30.0 mM to 40.0 mM, 0.1 mM to 30.0 mM, 0.5 mM to 30.0 mM, 1.0 mM to 30.0 mM, 5.0 mM to 30.0 mM, 10.0 mM to 30.0 mM, 25.0 mM to 30.0 mM, 0.1 mM to 20.0 mM, 0.5 mM to 20.0 mM, 1.0mM to 20.0 mM, 5.0 mM to 20.0 mM, 10.0 mM to 20.0 mM, 0.1 mM to 10.0 mM, 0.5 mM to 10.0 mM, 1.0 mM to 10.0 mM, or 5.0 mM to 10.0 mM. In a specific embodiment, the amino acid may be 17 mM to 29 mM of arginine, a pharmaceutically acceptable salt thereof, or a mixture thereof. In another specific embodiment, the amino acid may be 24 mM to 29 mM of glycine, a pharmaceutically acceptable salt thereof, or a mixture thereof. The concentration of the amino acid may be freely adjusted within the range in which the desired pH of the pharmaceutical formulation is not affected and the stability of an anti-PD-1 antibody, for example, a pembrolizumab, or an antigen binding fragment thereof, is not affected, and may individually vary depending on the specific type of the amino acid.

The metal salt may be NaCl, KCI, NaF, KBr, NaBr, Na₂SO₄, NaSCN, CaCl₂, MgCl₂, or K₂SO₄. The metal salt may be, for example, NaCl or Na₂SO₄. A concentration of the metal salt may be 1.0 mM to 300.0 mM, 5.0 mM to 150.0 mM, 10.0 mM to 150.0 mM, 30.0 mM to 150.0 mM, 50.0 mM to 150.0 mM, 80.0 mM to 150.0 mM, 100.0 mM to 150.0 mM, 120.0 mM to 150.0 mM, 5.0 mM to 125.0 mM, 10.0 mM to 125.0 mM, 30.0 mM to 125.0 mM, 50.0 mM to 125.0 mM, 80.0 mMto 125.0 mM, 100.0 mM to 125.0 mM, 120.0 mM to 125.0 mM, 5.0 mM to 100.0 mM, 10.0 mM to 100.0 mM, 30.0 mM to 100.0 mM, 50.0 mM to 100.0 mM, 80.0 mM to 100.0 mM, 5 mM to 80.0 mM, 10.0 mM to 80.0 mM, 30.0 mM to 80.0 mM, or 50.0 mM to 80.0 mM. In a specific embodiment, the metal salt may be sodium chloride in a concentration of 5.0 mM to 150.0 mM, 20.0 to 140.0 mM, or about 100.0 mM. The concentration of the metal salt may be freely adjusted within the range in which the stability of an anti-PD-1 antibody in the pharmaceutical formulation according to the present disclosure, for example, a pembrolizumab or an antigen binding fragment thereof, is maintained without precipitation, and may individually vary depending on the specific type of metal salts.

As used herein, the term "buffer" refers to a composition added to allow the pharmaceutical formulation to resist a change in pH. The buffer may act to maintain pH of the pharmaceutical formulation within a tolerable range. The buffer generally allows the pharmaceutical formulation to resist a change in pH through actions of acid-base conjugate components thereof. In this specification, when a concentration of buffer is mentioned, the mentioned concentration refers to a molarity of the buffer in the form of a free acid or a free base thereof.

In a specific embodiment, the buffer may be at least one selected from buffers that have buffering capacity. The buffer may be at least one selected from the group consisting of, for example, phosphoric acid, acetic acid, citric acid, succinic acid, carbonic acid, amino acid, ionic forms thereof, or pharmaceutically acceptable salts thereof. The amino acid may be histidine. The concentration of the amino acid may be freely adjusted within the range in which the desired pH of the liquid formulation is not affected and the stability of an antibody or an antigen binding fragment thereof is not affected, and may individually vary depending on the specific type of the amino acid. The salt may be an alkali metal salt or a hydrochloric acid salt. The alkali metal may be sodium or potassium.

The buffer may be at least one selected from the group consisting of, phosphoric acid, acetic acid, citric acid, succinic acid, carbonic acid, histidine, ionic forms thereof, or pharmaceutically acceptable salts thereof.

In another specific embodiment, the buffer may be at least one selected from acetic acid, acetate, succinic acid, succinate, histidine, phosphoric acid, phosphate, citric acid, citrate, carbonic acid, carbonate, and pharmaceutically acceptable salts thereof.

A concentration of the buffer may be 1 mM to 100 mM, 1 mM to 70 mM, 1 mM to 50 mM, 1 mM to 40 mM, 1 mM to 30 mM, 1 mM to 25 mM, 1 mM to 22 mM, 1 mM to 20 mM, 5 mM to 100 mM, 5 mM to 70 mM, 5 mM to 50 mM, 5 mM to 40 mM, 5 mM to 30 mM, 5 mM to 25 mM, 5 mM to 22 mM, 5 mM to 20 mM, 10 mM to 100 mM, 10 mM to 70 mM, 10 mM to 50 mM, 10 mM to 40 mM, 10 mM to 30 mM, 10 mM to 25 mM, 10 mM to 22 mM, 10 mM to 20 mM, 14 mM to 100 mM, 14 mM to 70 mM, 14 mM to 50 mM, 14 mM to 40 mM, 14 mM to 30 mM, 14 mM to 25 mM, 14 mM to 22 mM, 14 mM to 20 mM, 16 mM to 100 mM, 16 mM to 70 mM, 16 mM to 50 mM, 16 mM to 40 mM, 16 mM to 30 mM, 16 mM to 25 mM, 16 mM to 22 mM, or 16 mM to 20 mM, for example, 16 mM, 17 mM, 18 mM, 19 mM, or 20 mM.

The pharmaceutical formulation may not include a surfactant. The surfactant may be a non-ionic surfactant. The non-ionic surfactant may be polysorbate, poloxamer, sorbitan ester of another fatty acid, or a mixture thereof. The polysorbate may be polysorbate 20, polysorbate 40, polysorbate 60, polysorbate 80, or a mixture thereof. The poloxamer may be poloxamer 188.

The pharmaceutical formulation may be liquid. The pharmaceutical formulation may be administered by subcutaneous or intravenous injection. The pharmaceutical formulation may further include an appropriate aqueous carrier appropriate for the injection. The aqueous carrier is a safe, non-toxic, pharmaceutically acceptable carrier that may be administered to a human, and examples thereof may include water, a saline solution, a Ringer's solution, dextrose, or a mixture thereof.

The pharmaceutical formulation may have an osmotic pressure in a range appropriate for subcutaneous or intravenous injection. An osmotic pressure may be, for example, 200 mOsm/kg to 400 mOsm/kg, 200 mOsm/kg to 350 mOsm/kg, 250 mOsm/kg to 300 mOsm/kg, 250 mOsm/kg to 290 mOsm/kg, 270 mOsm/kg to 328 mOsm/kg, 250 mOsm/kg to 269 mOsm/kg, or 328 mOsm/kg to 350 mOsm/kg. The osmotic pressure may be appropriately adjusted to minimize a pain that may be caused when the pharmaceutical formulation is administered.

The pharmaceutical formulation may have viscosity in a range suitable for subcutaneous or intravenous injection. When the viscosity is measured at room temperature of 25±3 °C, the viscosity may be, for example, 0.5 cp to 100 cp, 0.5 cp to 90 cp, 0.5 cp to 80 cp, 0.5 cp to 70 cp, 0.5 cp to 60 cp, 0.5 cp to 50 cp, 0.5 cp to 40 cp, 0.5 cp to 30 cp, 0.5 cp to 20 cp, 0.5 cp to 15 cp, or 0.5 cp to 10 cp. The viscosity may be appropriately adjusted to minimize a pain that may be caused when the pharmaceutical formulation is administered.

In the pharmaceutical formulation according to a specific embodiment, the anti-PD-1 antibody, for example, a pembrolizumab, or the antigen binding fragment thereof, may be contained in a concentration of 5 mg/mL to 300 mg/mL, 5 mg/mL to 200 mg/mL, 15 mg/mL to 30 mg/mL, 140 mg/mL to 160 mg/mL, the stabilizer may be sucrose, glucose, galactose, maltose, fructose, trehalose, sorbitol, mannitol, arginine, lysine, proline, glycine, phenylalanine, tyrosine, tryptophan, a hydrate thereof, a pharmaceutically acceptable salt thereof, or a mixture thereof, and the pharmaceutical formulation may have a pH in the range of 4.5 to 6.5.

The stabilizer may be sucrose, glucose, galactose, maltose, fructose, trehalose, a hydrate thereof, or a mixture thereof, and a concentration of the stabilizer may be 1.0 %(w/v) to 15.0 %(w/v), 3.0 %(w/v) to 15.0 %(w/v), 5.0 %(w/v) to 15.0 %(w/v), 7.0 %(w/v) to 15.0 %(w/v), 1.0 %(w/v) to 10.0 %(w/v), 3.0 %(w/v) to 10.0 %(w/v), 5.0 %(w/v) to 10.0 %(w/v), 7.0 %(w/v) to 10.0 %(w/v), 1.0 %(w/v) to 7.0 %(w/v), 2.0 %(w/v) to 7.0 %(w/v), 3.0 %(w/v) to 7.0 %(w/v), 4.0 %(w/v) to 7.0 %(w/v), 1.0 %(w/v) to 5.0 %(w/v), 0.02 %(w/v) to 5.0 %(w/v), 3.0 to 5.0 %(w/v), 4.0 %(w/v) to 5.0 %(w/v), 4.5 %(w/v) to 5.0 %(w/v) (e.g., about 4.7 %(w/v)), or 7.8 %(w/v) to 8.2 %(w/v) (e.g., about 7.8 %(w/v), about 7.9 %(w/v), about 8 %(w/v), about 8.1 %(w/v), or about 8.2 %(w/v)). The stabilizer may be sorbitol, mannitol, a hydrate thereof, or a mixture thereof, and a concentration of the stabilizer may be 1.0 %(w/v) to 20.0 %(w/v), for example, 1.0 %(w/v) to 15.0 %(w/v), 1.0 %(w/v) to 10.0 %(w/v), 2.5 %(w/v) to 10.0 %(w/v), 3.0 %(w/v) to 10.0 %(w/v), 3.5 %(w/v) to 10.0 %(w/v), 4.0 %(w/v) to 10.0 %(w/v), 1.0 %(w/v) to 8.0 %(w/v), 2.5 %(w/v) to 8.0 %(w/v), 3.0 %(w/v) to 8.0 %(w/v), 3.5 %(w/v) to 8.0 %(w/v), 4.0 %(w/v) to 8.0 %(w/v), 1.0 %(w/v) to 6.0 %(w/v), 2.5 %(w/v) to 6.0 %(w/v), 3.0 %(w/v) to 6.0 %(w/v), 3.5 %(w/v) to 6.0 %(w/v), 4.0 t %(w/v) o 6.0 %(w/v), 4.0 %(w/v) to 5.5 %(w/v), or 4.0 %(w/v) to 5.0 %(w/v). The stabilizer may be arginine, lysine, proline, glycine, phenylalanine, tyrosine, tryptophan, a hydrate thereof, a pharmaceutically acceptable salt thereof, or a mixture thereof, and a concentration of the stabilizer may be 0.1 mM to 300.0 mM, 0.5 mM to 300.0 mM, 1.0 mM to 300.0 mM, 5.0 mM to 300.0 mM, 10.0 mM to 300.0 mM, 25.0 mM to 300.0 mM, 30.0 mM to 300.0 mM, 50.0 mM to 300.0 mM, 80.0 mM to 300.0 mM, 100.0 mM to 300.0 mM, 120.0 mM to 300.0 mM, 0.1 mM to 250.0 mM, 0.5 mM to 250.0 mM, 1.0 mM to 250.0 mM, 5.0 mM to 250.0 mM, 10.0 mM to 250.0 mM, 25.0 mM to 250.0 mM, 30.0 mM to 250.0 mM, 50.0 mM to 250.0 mM, 80.0 mM to 250.0 mM, 100.0 mM to 250.0 mM, 120.0 mM to 250.0 mM, 0.1 mM to 200.0 mM, 0.5 mM to 200.0 mM, 1.0 mM to 200.0 mM, 5.0 mM to 200.0 mM, 10.0 mM to 200.0 mM, 25.0 mM to 200.0 mM, 30.0 mM to 200.0 mM, 50.0 mM to 200.0 mM, 80.0 mM to 200.0 mM, 100.0 mM to 200.0 mM, 120.0 mM to 200.0 mM, 0.1 mM to 160.0 mM, 0.5 mM to 160.0 mM, 1.0 mM to 160.0 mM, 5.0 mM to 160.0 mM, 10.0 mM to 160.0 mM, 25.0 mM to 160.0 mM, 30.0 mM to 160.0 mM, 50.0 mM to 160.0 mM, 80.0 mM to 160.0 mM, 100.0 mM to 160.0 mM, 120.0 mM to 160.0 mM, 130.0 mM to 150.0 mM, 0.1 mM to 100.0 mM, 0.5 mM to 100.0 mM, 1.0 mM mM to 100.0 mM, 5.0 mM mM to 100.0 mM, 10.0 mM to 100.0 mM, 25.0 mM to 100.0 mM, 30.0 mM to 100.0 mM, 50.0 mM to 100.0 mM, 80.0 mM to 100.0 mM, 0.1 mM to 50.0 mM, 0.5 mM to 50.0 mM, 1.0 mM to 50.0 mM, 5.0 mM to 50.0 mM, 10.0 mM to 50.0 mM, 25.0 mM to 50.0 mM, 30.0 mM to 50.0 mM, 0.1 mM to 40.0 mM, 0.5 mM to 40.0 mM, 1.0 mM to 40.0 mM, 5.0 mM to 40.0 mM, 10.0 mM to 40.0 mM, 25.0 mM to 40.0 mM, 30.0 mM to 40.0 mM, 0.1 mM to 30.0 mM, 0.5 mM to 30.0 mM, 1.0 mM to 30.0 mM, 5.0 mM to 30.0 mM, 10.0 mM to 30.0 mM, 25.0 mM to 30.0 mM, 0.1 mM to 20.0 mM, 0.5 mM to 20.0 mM, 1.0 mM to 20.0 mM, 5.0 mM to 20.0 mM, 10.0 mM to 20.0 mM, 0.1 mM to 10.0 mM, 0.5 mM to 10.0 mM, 1.0 mM to 10.0 mM, or 5.0 mM to 10.0 mM.

In the specific embodiment, the buffer may include phosphoric acid, acetic acid, citric acid, succinic acid, carbonic acid, histidine, or pharmaceutically acceptable salts thereof. The salt may be an alkali metal salt or a hydrochloric acid salt. The alkali metal may be sodium or potassium.

In the specific embodiment, a concentration of the buffer may be 1 mM to 100 mM, 1 mM to 70 mM, 1 mM to 50 mM, 1 mM to 40 mM, 1 mM to 30 mM, 1 mM to 25 mM, 1 mM to 22 mM, 1 mM to 20 mM, 5 mM to 100 mM, 5 mM to 70 mM, 5 mM to 50 mM, 5 mM to 40 mM, 5 mM to 30 mM, 5 mM to 25 mM, 5 mM to 22 mM, 5 mM to 20 mM, 10 mM to 100 mM, 10 mM to 70 mM, 10 mM to 50 mM, 10 mM to 40 mM, 10 mM to 30 mM, 10 mM to 25 mM, 10 mM to 22 mM, 10 mM to 20 mM, 14 mM to 100 mM, 14 mM to 70 mM, 14 mM to 50 mM, 14 mM to 40 mM, 14 mM to 30 mM, 14 mM to 25 mM, 14 mM to 22 mM, 14 mM to 20 mM, 16 mM to 100 mM, 16 mM to 70 mM, 16 mM to 50 mM, 16 mM to 40 mM, 16 mM to 30 mM, 16 mM to 25 mM, 16 mM to 22 mM, or 16 mM to 20 mM, for example, 16 mM, 17 mM, 18 mM, 19 mM, or 20 mM.

In the pharmaceutical formulation of the present disclosure, the anti-PD-1 antibody, for example, a pembrolizumab, or the antigen binding fragment thereof, may be stabilized. The term "stabilization" may mean that an anti-PD-1 antibody, for example, a pembrolizumab, or an antigen binding fragment thereof, substantially retains its physical stability, chemical stability and/or biological activity before and after administration, and during additional manufacturing processes, preservation or storage. The physical stability, chemical stability and/or biological activity may be evaluated by commonly known methods.

In this specification, the stability of an anti-PD-1 antibody or an antigen binding fragment thereof may meet one or more of the following details.

The stability may be measured at a selected temperature for a selected time period. For example, in a specific embodiment, a stable formulation is a formulation in which no significant change is observed at 2 °C to 8 °C for 12 months or more. In another specific embodiment, a stable formulation is a formulation in which no significant change is observed at 2 °C to 8 °C for 18 months or more. In another specific embodiment, a stable formulation is a formulation in which no significant change is observed at 23 °C to 27 °C for 3 months or more. In another specific embodiment, a stable formulation is a formulation in which no significant change is observed at 23 °C to 27 °C for 6 months or more. In another specific embodiment, a stable formulation is a formulation in which no significant change is observed at 23 °C to 27 °C for 12 months or more. In another specific embodiment, a stable formulation is a formulation in which no significant change is observed at 23 °C to 27 °C for 18 months or more. The followings are stability criteria for an antibody formulation. When measured by size exclusion-high-performance liquid chromatography (SE-HPLC), 10% or less, for example, 5% or less, or 2.5% or less of antibody monomers is denatured. The antibody has a potency within a range of 60 % to 140 %, or 80 % to 120 % of that of a control group or a standard antibody. For example, when low molecular weight (LMW) species is measured by SE-HPLC, 10 % or less, 5 % or less, or 2.5 % or less of antibody shows a change. For example, when high molecular weight (HMW) species is measured by SE-HPLC, 10 % or less, 5 % or less, or 2.5 % or less of antibody shows a change. In addition, a concentration and pH of the formulation may be changed in a range of ±10 % or less, ±5 % or less, or ±2.5 % or less.

When the pharmaceutical formulation is placed in a stability thermostat and then stored under the conditions of a temperature of 40 ± 2 °C and a relative humidity of 75±5 % for 4 weeks, the HMW or pH of the pharmaceutical formulation is changed within a range of 10 % or less, 5 % or less, or 2.5 % or less.

When 0.3 mL to 1 mL of the pharmaceutical formulation is placed in a type I, 2 cc glass vial (Schott Inc.) and the vial is mounted on a stirrer (Heidolph Instruments) for stirring at room temperature at 400 rpm for 72 hours, the HMW or pH of the pharmaceutical formulation is changed within a range of 10 % or less, 5 % or less, or 2.5 % or less.

The pembrolizumab or the antigen binding fragment thereof retains its chemical stability in the pharmaceutical formulation in a case it is chemically stable in a predetermined time period in which the biological activity is considered to be retained. The chemical stability may be evaluated by detecting and quantifying chemically modified pembrolizumab. The chemical modification includes size modification or charge change. The charge change may be a change resulting from, for example, de-amidation. The size modification may be evaluated by size-exclusion high-performance liquid chromatography (SE-HPLC), sodium dodecyl sulfate-polyacrylamide gel electrophoresis (SDS-PAGE), capillary electrophoresis-sodium dodecyl sulfate (CE-SDS) assay, and matrix-assisted laser desorption/ionization/time-of-flight mass spectrometry (MALDI/TOF MS). In addition, the charge change may be evaluated by ion exchange chromatograph (IEC), and imaging capillary isoelectric focusing (icIEF). The activity may be determined by PD-1 ligand binding assay. The PD-1 ligand binding assay may be performed by enzyme-linked immunosorbent assay (ELISA). In the ELISA analysis, a relative binding rate (%) of pembrolizumab binding to PD-1 ligand is measured. A pembrolizumab sample is reacted with PD-1 ligand on ELISA plate, and an absorbance is measured at 450 nm to obtain a relative binding rate (%).

The anti-PD-1 antibody, for example, a pembrolizumab, or an antigen binding fragment thereof retains the biological activity in the pharmaceutical formulation. For example, when the biological activity of the anti-PD-1 antibody or the antigen binding fragment thereof in the pharmaceutical formulation is within about 30 %, about 20 %, or about 10 % (or within an analysis error range) of the biological activity exhibited at a manufacturing time point of the pharmaceutical formulation, the pharmaceutical formulation is considered to retain its biological activity. The biological activity may be determined by, for example, antigen binding assay.

The stability may be evaluated by measuring % HMW, % monomer and/or % LMW by using size-exclusion HPLC (SE-HPLC) after applying temperature stress, for example, at 40 °C for 1 week to 4 weeks, freeze-thaw stress, for example, by repeating a cycle of freezing at -70 °C and thawing at room temperature 5 times, or stirring stress, for example, by applying a rotational force at 400 rpm for 72 hours in a stirrer. In a specific embodiment, Δ%HMW, Δ%LMW, or Δ% monomer values of the pharmaceutical formulation may be equal to or smaller than those of Keytruda^{®}.

When the stable pharmaceutical formulation provided herein includes 25 mg/mL of an antibody (pH 5.5), a %HMW variance, that is, %HMW value at week 4 - %HMW value at week 0, may be 10.0 % or less, 5.0 % or less, or 2.5 %, as measured by general size exclusion chromatography (SEC) after 0.3 mL to 1 mL of the formulation is put into a type I, 2 cc glass vial (Schott Inc.) and then stored at 40 °C for 4 weeks.

Another aspect provides a method for treating a cancer using the pharmaceutical formulation. The pharmaceutical formulation is the same as described above. The method for treating a cancer may comprise administering the pharmaceutical formulation in a therapeutically effective amount to a subject. The subject may be a human.

In the method, the term "cancer" refers to or describes pathological conditions in mammals, typically characterized by up-regulated cell growth. The cancer includes carcinoma, lymphoma, leukemia, blastoma and sarcoma, but is not limited thereto. The cancer may include, for example, squamous cell carcinoma, myeloma, small cell lung cancer, non-small cell lung cancer, glioma, Hodgkin lymphoma, non-Hodgkin lymphoma, gastrointestinal cancer or gastrointestinal tract cancer, renal cancer, ovarian cancer, liver cancer, lymphoblastic leukemia, lymphocytic leukemia, colorectal cancer, endometrial cancer, kidney cancer, prostate cancer, thyroid cancer, melanoma, chondrosarcoma, neuroblastoma, pancreatic cancer, glioblastoma multiforme, cervical cancer, brain cancer, stomach cancer, bladder cancer, hepatoma, breast cancer, colon carcinoma, Merkel cell cancer, high microsatellite instability cancer, esophageal cancer, urothelial cancer, primary mediastinal large B-cell lymphoma, and head and neck cancer.

Another aspect provides a method for preparing a stable pharmaceutical formulation, comprising: preparing a mixed solution by adding a stabilizer to a solvent; and adding an anti-PD-1 antibody or an antigen binding fragment thereof to the mixed solution; or comprising: preparing a solution of an anti-PD-1 antibody or an antigen binding fragment thereof by adding the anti-PD-1 antibody or the antigen binding fragment thereof to a solvent; and adding a stabilizer to the solution, wherein the preparation method is performed without adding a buffer.

In the method, the solvent may be an aqueous solvent, for example, water or a saline solution. The method may further comprise adding a surfactant to the mixed solution having the anti-PD-1 antibody or the antigen binding fragment thereof added thereto.

Among the terms or elements mentioned in the pharmaceutical formulation, the same terms or elements as those mentioned in the description of the claimed method for preparing the pharmaceutical formulation should be understood to be the same as those mentioned previously in the description of the claimed pharmaceutical formulation.

### ADVANTAGEOUS EFFECTS OF DISCLOSURE

In a stable anti-PD-1 antibody pharmaceutical formulation according to an aspect, the anti-PD-1 antibody may be stably maintained even without having a surfactant.

In a method for treating a cancer in a subject according to an aspect, the cancer may be efficiently treated in the subject.

By a method for preparing a stable anti-PD-1 antibody pharmaceutical formulation according to an aspect, the stable anti-PD-1 antibody pharmaceutical formulation may be efficiently prepared.

### BRIEF DESCRIPTION OF DRAWINGS

FIG. 1 shows a T_{agg} value (temperature at which antibodies aggregate) of buffer-free formulations or formulations including different amounts of histidine buffer and surfactants; and
FIG. 2 shows Δ%HMW values of formulations including different amounts of buffers and PS80 after applying temperature stress thereto for 4 weeks.

### BEST MODE

Hereinafter, the present disclosure will be described in detail with reference to the following examples. However, the following examples are intended to illustrate the present disclosure, and the scope of the present disclosure is not limited thereto.

### Materials and Methods

### 1. Size Exclusion Chromatography (SEC)

The purity of each sample was identified by size-exclusion chromatography. Percentages of antibody monomer, high molecular weight species (HMW), and low molecular weight species (LMW) are determined by the size-exclusion chromatography. In the size-exclusion chromatography, LMW is eluted later than HMW. The presence of HMW indicates protein aggregates, and the presence of LMW indicates protein fragments.

### 2. Dynamic Light Scattering (DLS) Assay

Aggregates in each sample were measured by dynamic light scattering assay. Specifically, each sample was diluted using each buffer and loaded onto wells of a 96-well plate. The plate was loaded onto DynoPro^{®} Plate Reader^{™} II instrument (Wyatt Technology). The temperature of the sample was increased at a rate of 0.15 °C/min in a range of 25 °C to 70 °C, and sizes of aggregates in the pharmaceutical formulation were measured by dynamic light scattering (DLS) assay. Evaluation of stability is performed by measuring the temperature at a time point at which the aggregate size varies, and a higher T_{agg} value means a higher level of stability.

### 3. Preparation of Pharmaceutical Formulations

Various pharmaceutical formulations listed in Table 1 were prepared in the following manners and used in the following examples.

First, in the composition of each of the respective pharmaceutical formulations, a buffer and a stabilizer, except for pembrolizumab and a surfactant, were added to sterilized distilled water to prepare a buffer solution. Pembrolizumab was introduced into a dialysis cassette (Slide-A-Lyzer cassette, Thermo Fisher Scientific) and then placed in a beaker containing a solution of each of the compositions listed in Table 1 to perform dialysis, thereby exchanging the existing pembrolizumab solution with the buffer solution. Lastly, in the case of formulations including a surfactant, PS80 surfactant was added to the pembrolizumab solution so that the concentration of the surfactant in the prepared buffer solution reached a 1x concentration. Thereafter, the concentration was adjusted using each solution so that the concentration of pembrolizumab finally reached 25 mg/mL.

**[Table 1]**

| Name | Group | Formulation composition |
|---|---|---|
| 1A | 1 (surfactant free) | Surfactant free, 0 mM histidine, 7.0 % sucrose |
| 1B | | Surfactant free, 20 mM histidine, 7.0 % sucrose |
| 1C | | Surfactant free, 40 mM histidine, 7.0 % sucrose |
| 2A | 2 (0.02% PS80) | 0.02 % Surfactant, 0 mM histidine, 7.0 % sucrose |
| 2B | | 0.02 % Surfactant, 20 mM histidine, 7.0 % sucrose |
| 2C | | 0.02 % Surfactant, 40 mM histidine, 7.0 % sucrose |
| 3A | 3 (0.1% PS80) | 0.10 % Surfactant, 0 mM histidine, 7.0 % sucrose |
| 3B | | 0.10 % Surfactant, 20 mM histidine, 7.0 % sucrose |
| 3C | | 0.10 % Surfactant, 40 mM histidine, 7.0 % sucrose |
| 4A | 4 (0.2% PS80) | 0.20 % Surfactant, 0 mM histidine, 7.0 % sucrose |
| 4B | | 0.20 % Surfactant, 20 mM histidine, 7.0 % sucrose |
| 4C | | 0.20 % Surfactant, 40 mM histidine, 7.0 % sucrose |

In table 1, in groups 2 to 4, the surfactant is PS80, that is, polysorbate 80.

### Example 1: Effect of Different Amounts of Buffer and Surfactant on Stability of Pembrolizumab-Containing Pharmaceutical Formulation

In this example, the effect of a buffer and a surfactant on the stability of a pembrolizumab-containing pharmaceutical formulation was identified. Specifically, T_{agg} values of formulations 1A, 1B, 1C, 2A, 2B, 2C, 3A, 3B, 3C, 4A, 4B, and 4C were measured by dynamic light scattering (DLS) assay.

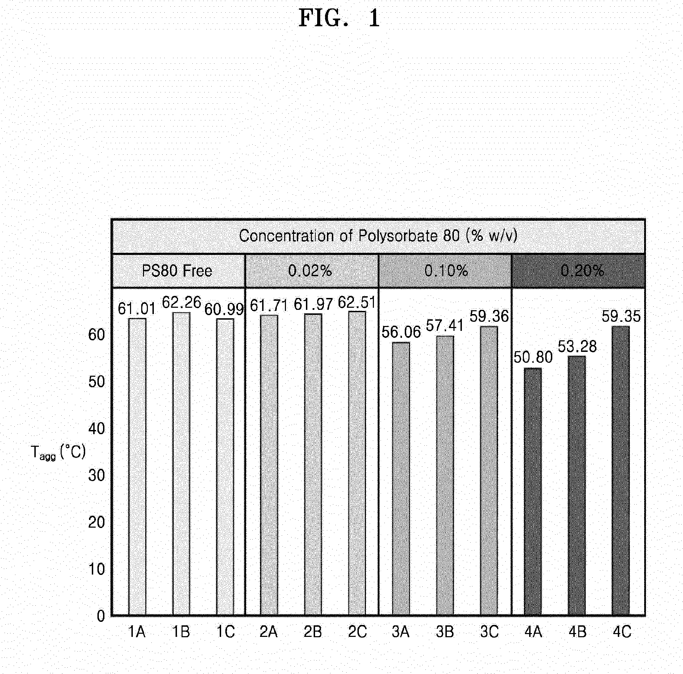

The results are shown in Table 2 and FIG. 1. Table 2 shows compositions of formulations having different amounts of a PS80 surfactant and a histidine buffer and T_{agg} values measured therefrom. FIG. 1 shows T_{agg} values of formulations including different amounts of a surfactant and a histidine buffer.

**[Table 2]**

| Name | Formulation composition | T_{agg} (°C) |
|---|---|---|
| 1A | Surfactant free, 0 mM histidine, 7.0 % sucrose | 61.01 |
| 1B | Surfactant free, 20 mM histidine, 7.0 % sucrose | 62.26 |
| 1C | Surfactant free, 40 mM histidine, 7.0 % sucrose | 60.99 |
| 2A | 0.02 % Surfactant, 0 mM histidine, 7.0 % sucrose | 61.71 |
| 2B | 0.02 % Surfactant, 20 mM histidine, 7.0 % sucrose | 61.97 |
| 2C | 0.02 % Surfactant, 40 mM histidine, 7.0 % sucrose | 62.51 |
| 3A | 0.10 % Surfactant, 0 mM histidine, 7.0 % sucrose | 56.06 |
| 3B | 0.10 % Surfactant, 20 mM histidine, 7.0 % sucrose | 51.41 |
| 3C | 0.10 % Surfactant, 40 mM histidine, 7.0 % sucrose | 59.36 |
| 4A | 0.20 % Surfactant, 0 mM histidine, 7.0 % sucrose | 50.80 |
| 4B | 0.20 % Surfactant, 20 mM histidine, 7.0 % sucrose | 53.28 |
| 4C | 0.20 % Surfactant, 40 mM histidine, 7.0 % sucrose | 59.35 |

As shown in Table 2 and FIG. 1, the T_{agg} values of formulations that do not include a PS80 surfactant or formulations including PS80 in an amount of 0.02 % were higher than T_{agg} values of formulations including PS80 in an amount of 0.10 % or 0.20 %. This indicates that the formulations including PS80 in an amount less than 0.10 %, i.e., the formulations 1A, 1B, 1C, 2A, 2B, and 2C, have a higher temperature of aggregation than the formulations including PS80 in an amount of 0.10 % or more, i.e., the formulations 3A, 3B, 3C, 4A, 4B, and 4C. That is, formulations including less than 0.1 % of PS80 are more stable than formulations including 0.10 % or more of PS80. Meanwhile, in formulations having PS80 concentrations of less than 0.10 %, amounts of histidine did not significantly affect T_{agg} variation, as shown when comparing 1B with 1C, and 2B with 2C.

In addition, among formulations including 20 mM of histidine buffer, T_{agg} value of 1B which does not include PS80 was the highest and higher than T_{agg} values of formulations that include PS80, i.e., 2B, 3B, and 4B. On the other hand, among formulations including 40 mM of histidine buffer, T_{agg} value of a formulation including 0.02% of PS80, i.e., 2C was higher than T_{agg} values of 1C, 3C, and 4C.

### Example 2: Effect of Different amounts of Buffer and Surfactant on Stability of Pembrolizumab-Containing Pharmaceutical Formulation

In this example, the effect of a buffer and a surfactant on the stability of a pembrolizumab-containing pharmaceutical formulation was identified. Specifically, after applying temperature stress to formulations 1A, 1B, 1C, 2A, 2B, 2C, 3A, 3B, 3C, 4A, 4B, and 4C, purities of the formulation samples were measured by SEC.

The temperature stress was applied in the manner that 0.3 mL of each of the formulations was put into a type I, 2 cc glass vial and the vial was exposed to a 40 °C temperature stress condition for 4 weeks in a stability thermostat (JEIO TECH Co., Ltd.). Specifically, the vial was placed in the stability thermostat and maintained under the conditions of a temperature of 40±2 °C and a relative humidity of 75±5 % for 4 weeks. SEC analysis was performed on the formulations stored for 4 weeks in the above-described manner.

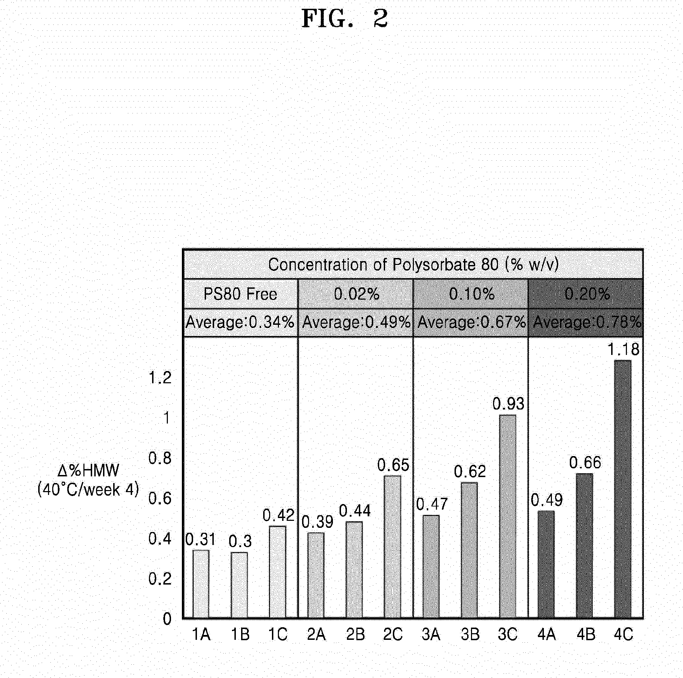

The results are shown in Table 3 and FIG. 2. Table 3 shows compositions of formulations including different amounts of a buffer and PS80, and purities of the formulations after applying temperature stress thereto for 4 weeks, as indicated by %HMW. FIG. 2 shows Δ%HMW values of formulations including different amounts of buffers and PS80 after applying temperature stress thereto for 4 weeks.

**[Table 3]**

| Nam e | Formulation composition | Week 0 | Week 4 | |
|---|---|---|---|---|
| | | %HM W | %HM W | △%HM W |
| 1A | Surfactant free, 0 mM histidine, 7.0 % sucrose | 0.19 | 0.50 | 0.31 |
| 1B | Surfactant free, 20 mM histidine, 7.0 % sucrose | 0.18 | 0.48 | 0.30 |
| 1C | Surfactant free, 40 mM histidine, 7.0 % sucrose | 0.18 | 0.60 | 0.42 |
| 2A | 0.02 % Surfactant, 0 mM histidine, 7.0 % sucrose | 0.20 | 0.59 | 0.39 |
| 2B | 0.02 % Surfactant, 20 mM histidine, 7.0 % sucrose | 0.18 | 0.62 | 0.44 |
| 2C | 0.02 % Surfactant, 40 mM histidine, 7.0 % sucrose | 0.19 | 0.84 | 0.65 |
| 3A | 0.10 % Surfactant, 0 mM histidine, 7.0 % sucrose | 0.28 | 0.75 | 0.47 |
| 3B | 0.10 % Surfactant, 20 mM histidine, 7.0 % sucrose | 0.25 | 0.87 | 0.62 |
| 3C | 0.10 % Surfactant, 40 mM histidine, 7.0 % sucrose | 0.27 | 1.20 | 0.93 |
| 4A | 0.20 % Surfactant, 0 mM histidine, 7.0 % sucrose | 0.41 | 0.90 | 0.49 |
| 4B | 0.20 % Surfactant, 20 mM histidine, 7.0 % sucrose | 0.39 | 1.05 | 0.66 |
| 4C | 0.20 % Surfactant, 40 mM histidine, 7.0 % sucrose | 0.41 | 1.59 | 1.18 |

As shown in Table 3 and FIG. 2, among the buffer-free formulations, △%HMW value of 1A which does not include PS80 was the lowest and lower than △%HMW value of formulations including PS80, i.e., 2A, 3A, and 4A. Among formulations including 20 mM of histidine buffer, △%HMW value of 1B which does not include PS80 was the lowest and lower than △%HMW value of formulations including PS80, i.e., 2B, 3B, and 4B. In addition, among the formulations including 40 mM of histidine buffer, △%HMW value of 1C which does not include PS80 was the lowest and lower than △%HMW values of formulations that include PS80, i.e., 2C, 3C, and 4C. This indicates that formulations of group 1 (1A, 1B, and 1C) which do not include a PS80 surfactant are more stable that formulations of group 2, 3, or 4 which include PS80.

In addition, pH of the formulations stored under a stress condition for 4 weeks was measured. Table 4 shows the results of measuring pH of samples left for 4 weeks under a stress condition of a temperature of 40 ° C.

**[Table 4]**

| Name | Formulation composition | pH | |
|---|---|---|---|
| | | 40°C/wee k 0 | 40°C/week 4 |
| 1A | Surfactant free, 0 mM histidine, 7.0 % sucrose | 5.50 | 5.57 |
| 1B | Surfactant free, 20 mM histidine, 7.0 % sucrose | 5.59 | 5.55 |
| 1C | Surfactant free, 40 mM histidine, 7.0 % sucrose | 5.56 | 5.52 |
| 2A | 0.02 % Surfactant, 0 mM histidine, 7.0 % sucrose | 5.47 | 5.60 |
| 2B | 0.02 % Surfactant, 20 mM histidine, 7.0 % sucrose | 5.57 | 5.53 |
| 2C | 0.02 % Surfactant, 40 mM histidine, 7.0 % sucrose | 5.54 | 5.51 |
| 3A | 0.10 % Surfactant, 0 mM histidine, 7.0 % sucrose | 5.46 | 5.53 |
| 3B | 0.10 % Surfactant, 20 mM histidine, 7.0 % sucrose | 5.58 | 5.54 |
| 3C | 0.10 % Surfactant, 40 mM histidine, 7.0 % sucrose | 5.55 | 5.50 |
| 4A | 0.20 % Surfactant, 0 mM histidine, 7.0 % sucrose | 5.49 | 5.55 |
| 4B | 0.20 % Surfactant, 20 mM histidine, 7.0 % sucrose | 5.64 | 5.52 |
| 4C | 0.20 % Surfactant, 40 mM histidine, 7.0 % sucrose | 5.53 | 5.51 |

As shown in Table 4, when pH of formulations 1A, 1B and 1C which do not include polysorbate 80 are compared with pH of formulations 2A, 2B, 2C, 3A, 3B, 3C, 4A, 4B, and 4C which include 0.02% to 0.20% PS80, no significant difference was observed. It was confirmed that pH stability was maintained in a formulation which does not include a surfactant as in a formulation which includes a surfactant.

## Claims

1. A stable anti-PD-1 antibody pharmaceutical formulation, comprising:
(a) an anti-PD-1 antibody or an antigen binding fragment thereof;
(b) a stabilizer; and
(c) a buffer,
wherein the formulation does not comprise a surfactant and has a pH of about 4.5 to about 6.5.

2. The pharmaceutical formulation of claim 1, wherein the anti-PD-1 antibody is a pembrolizumab.

3. The pharmaceutical formulation of claim 1, wherein a concentration of the anti-PD-1 antibody or the antigen binding fragment thereof is about 5 mg/mL to about 300 mg/mL.

4. The pharmaceutical formulation of claim 1, wherein a concentration of the pembrolizumab or the antigen binding fragment thereof is about 10 mg/mL to about 165 mg/mL.

5. The pharmaceutical formulation of claim 1, wherein a concentration of the anti-PD-1 antibody or the antigen binding fragment thereof is about 25 mg/mL or about 150 mg/mL.

6. The pharmaceutical formulation of claim 1, wherein the stabilizer is a polyol, an amino acid or a pharmaceutically acceptable salt thereof, or a mixture thereof.

7. The pharmaceutical formulation of claim 6, wherein the polyol is sorbitol, sucrose, trehalose, mannose, maltose, mannitol, or a mixture thereof.

8. The pharmaceutical formulation of claim 6, wherein the polyol is sorbitol, sucrose, trehalose, or a mixture thereof.

9. The pharmaceutical formulation of claim 6, wherein the amino acid is glycine, proline, phenylalanine, tyrosine, tryptophan, lysine, arginine, a pharmaceutically acceptable salt thereof, or a mixture thereof.

10. The pharmaceutical formulation of claim 1, wherein the stabilizer is about 1.0% (w/v) to about 15.0% (w/v) of a sugar, about 1.0% (w/v) to about 20.0% (w/v) of a sugar alcohol, about 0.1 mM to about 300.0 mM of an amino acid, or about 1.0 mM to about 300.0 mM of a metal salt.

11. The pharmaceutical formulation of claim 1, wherein the surfactant is a non-ionic surfactant.

12. The pharmaceutical formulation of claim 11, wherein the surfactant is polysorbate, poloxamer, sorbitan ester of another fatty acid, or a mixture thereof.

13. The pharmaceutical formulation of claim 12, wherein the polysorbate is polysorbate 20, polysorbate 80, or a mixture thereof.

14. The pharmaceutical formulation of claim 1, wherein the buffer is histidine, phosphoric acid, maleic acid, tartaric acid, succinic acid, citric acid, acetic acid, carbonic acid, a pharmaceutically acceptable salt thereof, or a mixture thereof.

15. The pharmaceutical formulation of claim 1, wherein a concentration of the buffer is about 5 mM to less than about 40 mM.

16. The pharmaceutical formulation of claim 1, wherein a concentration of the anti-PD-1 antibody or the antigen binding fragment thereof is about 5 mg/mL to about 300 mg/mL, the stabilizer is about 1.0% (w/v) to about 15.0% (w/v) of sucrose, trehalose, a hydrate thereof, or a mixture thereof, about 1.0% (w/v) to about 20.0% (w/v) of sorbitol, mannitol, a hydrate thereof, or a mixture thereof, or about 0.1 mM to about 300.0 mM of arginine, lysine, proline, glycine, phenylalanine, tyrosine, tryptophan, a pharmaceutically acceptable salt thereof, or a mixture thereof, and the buffer is about 5 mM to about less than 40 mM of histidine, phosphoric acid, maleic acid, tartaric acid, succinic acid, citric acid, acetic acid, carbonic acid, a pharmaceutically acceptable salt thereof, or a mixture thereof.

17. The pharmaceutical formulation of claim 16, wherein the concentration of the anti-PD-1 antibody or the antigen binding fragment thereof is about 5 mg/mL to about 200 mg/mL.

18. The pharmaceutical formulation of claim 16, wherein the concentration of the anti-PD-1 antibody or the antigen binding fragment thereof is about 15 mg/mL to about 30 mg/mL, or about 140 mg/mL to about 160 mg/mL.

19. The pharmaceutical formulation of claim 16, wherein the pharmaceutical formulation does not comprise a non-ionic surfactant.

20. The pharmaceutical formulation of claim 19, wherein the surfactant is polysorbate, poloxamer, sorbitan ester of another fatty acid, or a mixture thereof.

21. A method for treating a cancer in a subject, comprising administering a therapeutically effective amount of the pharmaceutical formulation according to claim 1 to the subject.

22. A method for preparing a stable anti-PD-1 antibody pharmaceutical formulation, comprising:
preparing a mixed solution by adding a stabilizer and a buffer to a solvent, and
adding pembrolizumab or an antigen binding fragments thereof to the mixed solution; or
preparing a solution of pembrolizumab or an antigen binding fragments thereof by adding pembrolizumab or the antigen binding fragments thereof to a solvent, and
adding a stabilizer and a buffer to the solvent.
